# EUROPÄISCHE PATENTSCHRIFT

(11) **EP 1 383 556 B1**
(45) Veröffentlichungstag und Bekanntmachung des Hinweises auf die Patenterteilung: **24.10.2007**
(21) Anmeldenummer: 02737976.7
(22) Anmeldetag: 21.04.2002
(51) Int. Cl.: A61M 5/00, A61K 39/00

(54) **INJEKTIONSGERÄT FÜR mRNA APPLIKATION**
INJECTION DEVICE FOR ADMINISTERING mRNA
APPAREIL D'INJECTION POUR ADMINISTRATION DE mARN

(30) Priorität: 21.04.2001 DE 10119568
(43) Veröffentlichungstag der Anmeldung: 28.01.2004
(73) Patentinhaber: Curevac GmbH, 72076 Tübingen (DE)
(72) Erfinder: HOERR, Ingmar, 72070 Tübingen (DE); PASCOLO, Steve, 72070 Tübingen (DE); VON DER MüLBE, Florian, 72070 Tübingen (DE)
(74) Vertreter: Graf von Stosch, Andreas
(86) Internationale Anmeldenummer: PCT/EP2002/004380
(87) Internationale Veröffentlichungsnummer: WO 2002/085434

(56) Entgegenhaltungen:
- EP-A- 1 074 625
- EP-A- 1 083 232
- US-A- 3 052 239
- US-A- 4 306 554
- US-A- 5 429 603

## Beschreibung

Die Erfindung betrifft eine Vorrichtung zur intracutanen oder intradermalen Applikation getrockneter mRNA in einen Organismus insbesondere zum Zwecke der Immunisierung.

Impfungen gegen Erreger gehören seit vielen Jahrzehnten zu den breitesten und profiliertesten Formen der Gesundheitsvorsorge und werden auch weiterhin vielfach von Medizinem und Organisationen wie der WHO empfohlen. Gerade diese breite Anwendung bietet aber Anlaß zur intensiven Suche nach neuen, effektiven und kostengünstigen Lösungen auf diesem medizinischen Fachgebiet, das durch neue Impfstoffe (Vakzine), neue Anwendungsgebiete und neue Applikationsformen ständig wächst und erweitert wird.

Insbesondere bei den Impfstoffen zeichnet sich eine neue Richtung und der wachsende Einfluß der Biotechnologie ab. Während üblicherweise die Impfung mit abgetöten Erregern, Teilen des Erregers, Stoffwechselprodukten u.ä. stattfand und stattfindet, gibt es eine Neuentwicklung auf dem Gebiet der Vakzin-Forschung, den direkten Gen Transfer in Form von Fremd-DNA (Desoxyribonukeinsäuren) oder Fremd-RNA (Ribonukleinsäuren). Hierbei wird die genetische Information der DNA transkribiert und dann z.B. zu einem Peptid oder Protein als Antigen, bspw. einem tumorspezifischen Antigen, translatiert. Gegen derartig translatierte Peptide oder Proteine wird schließlich eine (oder vergleichsweise stark erhöhte) Immunantwort hervorgerufen. Ein Vorteil bei dieser genetischen Immunisierung besteht in der einfachen Präparation dieser Nukleinsäuren in großer Reinheit der Menge sowie die Möglichkeit virale und Tumorerkrankungen zu therapieren und Prophylaxe zu betreiben.

Dabei wird im allgemeinen der Impfung mit DNA der Vorzug gegeben. RNA ist im Vergleich zu DNA und Proteinen sehr instabil. Die Instabilität wird hauptsächlich durch allgegenwärtige Kontamination mit RNasen (RNA abbauende Enzyme) sowie molekülspezifische Hydrolyseeigenschaften verursacht. Diesen Eigenschaften von RNA muß durch komplizierte Lagerungshaltung in Lösung bei -70°C sowie aufwendigen Verfahren bei Transport und Handhabung Rechnung getragen werden.

Das ist bedauerlich, da die Immunisierung mit RNA als Impfstoff als wesentlich sicherer einzustufen ist als mit DNA, insbesondere, wenn man nichtinfektiöse, nicht-replikative mRNA verwendet RNA birgt nicht die Gefahr, stabil in das chromosomale Genom zu integrieren. Auch ist sie wesentlich einfacher *in vivo* abbaubar. Somit wäre bei der genetischen Immunisierung RNA der Impfstoff der Wahl. Allerdings fehlt es wegen der oben genannten Instabilität und Lagerungsprobleme bisher an einer geeigneten Möglichkeit - sei es durch ein Verfahren oder ein geeignetes Gerät - die effektive Anwendung von RNA bei einer Impfung zu ermöglichen.

Impfungen werden in der Regel durch Applikation einer Vakzine/eines Impfstoffs In Muskelgewebe (intramuskulär. i.m.), in die Haut (intradermal, i.d.) oder unter die Haut (subcutan, s.c.) vollzogen. Die ersten genetischen Immunisierungen erfolgten intramuskulär, während erst später die Haut Immunisierung dazukam. Für den Einsatz von RNA erweist sich das Hautgewebe als bevorzugter Applikationsort. Hautgewebe unterscheidet sich von Muskelgewebe unter anderem dadurch, daß es reichhaltig mit Zellen der körpereigenen Abwehr durchsetzt ist Die Haut stellt nicht nur eine anatomische Barriere gegen eindringende Keime dar sondern auch eine immunologische Barriere. In die Haut eindringende Mikroorganismen mit ihren verschiedenen Fremdstrukturen inkl. Eiweiß und DNA werden hier von spezialisierten Zellen angegriffen, abgebaut und mit dem lymphatischen Abfluß in Lymphknoten transportiert, wo es zu Induktion von spezifischen Immunabwehrreaktionen kommt.

Während die intramuskuläre Applikation von DNA mit einer Kanüle erfolgen kann, wird bei der Transfektion der Haut mit DNA immer häufiger die Gene-Gun (Genpistole) als Methode angewendet. Ein Beispiel ist die PowderJect Technologie der PowderJect Pharmaceuticals plc., deren Anwendung bisher nur mit DNA beschrieben ist Hierbei müssen Jedoch Gold Partikel verwendet werden, die mit DNA beschichtet werden. Hierbei handelt es sich um ein teueres und - gerade für DNA - arbeitsintensives Verfahren.

Es sind außerdem eine Vielzahl von weiteren Verfahren zur Einführung von Nukleinsäuren in Organismen (Lipofektion von RNA, Injektion von nackten DNA-Plasmiden, Kopplung von gelöster RNA an polykationische Moleküle sowie Lipofektion von DNA) und eukaryontische Zellen (Calciumphosphat Transfektion, Polybren-Transfektion, Protoplasten-Fusion, Elektroporation, Lipofektion und Mikroinjektion) bekannt. So beschreibt EP 1 074 625 eine Vakzin-Zusammensetzung, die zum Schutz von Katzen gegen den Katzen-Immundeffizienz-Virus entweder durch intramuskuläre oder intradermale Injektion verabreicht werden könnte. EP 1 083 232 offenbart ein Verfahren zum Transfer von RNA, insbesondere mRNA, in Zellen und Organismen, wobei die RNA in Form eines Komplexes zwischen mindestens einer RNA und mindestens einem kationischen, insbesondere palykationischen, Peptid oder Protein in die Zelle bzw. den Organismus eingebracht wird. US 5,679,647 beschreibt schließlich Verfahren und Vorrichtungen zur Immunisierung gegen Antigene, insbesondere zur Immunisierung gegen Tumor-assoziierte Antigene, durch Verabreichung nackter Polynukleotide. Keines dieser Verfahren offenbart aber eine Vorrichtung zur Applikation getrockneter mRNA. Da sich RNA in Lösung als sehr instabil erweist, kann aber durch die genannten Methoden RNA nich nur sehr ineffizient oder aber als jeweils nur ganz frisch vor der Verabreichung zu präparierende Lösung als Impfstoff zum Einsatz kommen, so daß es - wie oben bereits festgestellt - immer noch an einer effektiven Methode zur Verabreichung von RNA, die auch im klinischen Alltag eingesetzt werden könnte, fehlt.

Der Erfindung liegt daher die Aufgabe zu Grunde, eine gegenüber dem Stand der Technik verbesserte Möglichkeit für den Einsatz von mRNA bei intracutanen oder intradermaler. Anwendung zu bieten, bzw. eine Möglichkeit zu finden, die Wirksamkeit von genetischen Impfungen zu verbessern.

Daher ist Gegenstand der Erfindung eine Vorrichtung zur intracutanen oder intradermalen Applikation getrockneter mRNA in einen Organismus, bei dem die Vorrichtung mit mindestens einer, vorzugsweise mehreren Hauptpenetrationsvorrichtung(en) ausgestaltet wobei dehydratisierte (im allgemeinen Sprachgabrauch auch häufig "dehydriert" bezeichnet), vorzugsweise getnergetrocknete, mRNA auf der (den) Hauptpenetrations vorrichtung(en) aufgebracht ist. Insbesondere ist die Vorrichtung eine Vakzinierungsvorrichtung.

Diese Vorrichtung erlaubt effektiv und auf einfach handhabbare Weise, mRNA - insbesondere zur Immunisierung- einzusetzen.

Dabei wird durch den Entzug von Wasser (die Dehydratisierung oder "Dehydrierung", s.o.) die Stabilität des Impfstoffes und damit die Wirkung der Impfung überhaupt verbessert. Im trockenen Zustand ist die mRNA nicht mehr durch RNasen angreifbar und es kann keine molekülspezifische Hydrolyse stattfinden. Die Dehydratisierung kann durch einen Gefriertrocknungsprozeß ("Lyophilisierung") im Vakuum, ggf. auch durch Sublimationstrocknung, oder durch einen Vakuumtrocknungsprozeß bei Raumtemperatur erfolgen, Prozesse, die dem Fachmann geläufig sind. Beispiele für entsprechende Prozesse finden sich beispielsweise in der WO 95/27721 oder im Artikel von Tsuka et al. In ANALYTICAL BIOCHEMISTRY 247, 458-461 (1997). Dabei kann die gelöste RNA auf ein Medium bzw. eine Matrix aufgebracht werden, welche positiv, negativ oder neutral geladen sein kann. Auch dieses mit mRNA in Lösungsmittel beladene Medium/ die Matrix wird dann einem Dehydratisierungsprozeß unterzogen. Die getrocknete mRNA kann von diesem Medium/dieser Matrix anschließend wieder in Lösung gebracht werden. Je nach Eigenschaften der mRNA (Länge und Struktur) hängt das Lösungsverhalten von den Ladungseigenschaften des Mediums/der Matrix sowie von der Art des Lösungsmittels ab. Beispiele für die Matrix, das Medium wären z. B. Protamin und Chitosan (positive Ladung). Zellulose (neutrale Ladung) oder Metalle (negative Ladung). Dabei sind Matrix, Medium und Träger als synonym zu betrachten und austauschbar. Matrix, Medium und Träger sind spezifisch ein gegen ein Lösungsmittel. Puffer inertes Trägermaterial, auf dem die mRNA nicht-kovalent aufgetragen und -später mit einem wässrigen Lösungsmittel wieder ablösbar- gebunden ist.

Dabei versteht man unter dehydratisiert ("dehydriert") bzw. getrocknet insbesondere einen Wassergehalt des Materials von ≤ 2% w/w. Die Begriffe dehydratisiert und getrocknet sind als synonym und austauschbar zu betrachten und bedeuten beide auch "in einem dehydratisierten Zustand stabilisiert".

Unter dem Begriff mRNA massenger (RiboNucleic Acid) versteht man eine in vitro transkribierte mRNA oder eine chemisch synthetisierte mRNA, wobei die genannten mRNA-Moleküle chemisch modifiziert sein können. Beispiele für die Modifikation sind insbesondere Veränderungen oder Ersetzen des Ribose-Rückgrates, beispielsweise durch Ersetzen durch Phosphorothioate oder durch Einsatz von LNAs (locked nucleic acids).

In einer besonders bevorzugten Form der Erfindung ist die dehydratisierte mRNA nichtinfektiös und/oder nicht-replikativ.

Dabei versteht man unter nichtinfektiös, daß die mRNA nicht selbst infektiös ist, also beispielsweise keine noch infektiöse Viren mRNA ist. In einer alternativen Ausgestaltung kann es sich bei der eingesetzten mRNA jedoch auch um eine therapeutische, aber nicht als Vakzine wirkende, dehydratisierte mRNA handeln, bspw. um eine "antisense mRNA" (die komplementär zu intrazellulärer mRNA sein kann und deren Translation blockiert) handeln.

Besonders bevorzugt ist eine erfindungsgemäße Vorrichtung, in der die mRNA nicht mit anderen Molekülen komplexiert ist. Dabei versteht man unter komplexiert die kovalente und nicht kovalente Bindung an ein anderes organisches Molekül, beispielsweise ein Protein.

Bevorzugt ist weiter eine erfindungsgemäße Vorrichtung, in der die dehydratisierte mRNA, vorzugsweise zum Zeitpunkt der intracutanen oder intradermalen Applikation getrockneter mRNA in den Organismus, nicht auf einer Matrix, insbesondere nicht auf Mikropartikel, aufgebracht ist Dabei betrachtet man unter Mikropartikel eine Form der Matrix (s.o.), also beispielsweise Metall- oder Polymerpartikel eines Durchmessers Im Bereich von ca. 1 µm oder kleiner.

In der erfindungsgemäßen Vorrichtung kann die dehydratisierte mRNA zum Zeitpunkt der intracutanen oder intradermalen Applikation getrockneter mRNA in den Organismus gelöst vorliegen.

Die erfindungsgemäße Vorrichtung kann ein internes Pufferreservoir (3), gegebenenfalls enthaltend einen Puffer, aufweisen

Besonders bevorzugt ist eine erfindungsgemäße Vorrichtung, die In allen Bereichen, die mit der dehydratisierten mRNA in Kontakt kommen, vorzugsweise vollständig, frei von Kontaminationen, insbesondere mit RNasen ist und/oder RNase-freies Material verwendet wird. Alternativ oder kumulativ können auch RNAse-Inhibitoren hinzugefügt werden, die etwaige RNAse-Rückstände oder-Verunreinigungen in ihrer Wirkung blockieren können.

in der erfindungsgemäßen Vorrichtung kann der in der Vorrichtung enthaltene bzw. zur Lösung der dehydratisierten RNA zum Zeitpunkt der intracutanen oder intradermalen Applikation getrockneter mRNA in den Organismus verwendete Puffer gute Transfektionseigenschaften aufweisen und sich durch eine hohe Löslichkeit für mRNA auszeichnen vorzugsweise ein Puffer mit 150 mM NaCl, 10 mM HEPES, pH 7,4 sein.

Die erfindungsgemäße Vorrichtung kann in einer Schutzhülle verpackt sein und/oder eine RNase-freie Schutzhülle (9) kann auf die Injektionsnadel (6) aufgebracht werden sein.

In der erfindungsgemäßen Vorrichtung kann die dehydratisierte mRNA in einem vom Pufferreservoir (3) getrennten Raum (10), beispielsweise dem Impfstofflösungsraum, vorliegen wobei diese Trennung aufhebbar ist.

In der erfindungsgemäßen Vorrichtung mit gelöster mRNA kann die dehydratisierte mRNA vor der Lösung in einem vom Pufferreservoir (3) getrennten Raum (10), beispielsweise dem Impfstofflösungsraum, vorliegen wobei diese Trennung aufhebbar ist, und der Raum (10), beispielsweise der Impfstofflösungsraum, nach der Lösung der RNA nicht mehr vom Pufferreservoir (3) getrennt ist.

Die erfindungsgemäße Vorrichtung kann eine Vorrichtung zur Abgabe geringster Mengen (8), vorzugsweise einen Flüssigkeitssammler, aufweisen

in der erfindungsgemäßen Vorrichtung können alle Öffnungen, durch die Luft oder Flüssigkeit in die Vorrichtung treten kann (1) oder (6), beispielsweise ein Ventil (1), mit Filtern, vorzugsweise Luftfiltern, vor Kontamination geschütztsein.

In der erfindungsgemäßen Vorrichtung kann das Pufferreservoir (3) eine durch ein vorgesehenes Öffnungsmittel (7) zu öffnende Stelle (4) aufweisen.

Es kann weiterhin die mRNA in der Vorrichtung direkt in der Vorrichtung, dem Injektionsgerät, in stabilisierter Form vorliegen. Diese applikationsfertige mRNA kann über einen längeren Zeitraum problemlos gelagert werden. Zu diesem Zweck wird dehydratisierte mRNA üblicherweise über eine Matrix(5) im Injektionsgerät aufbewahrt. Vor der Applikation wird allgemein die mRNA mit einem Injektionspuffer versetzt und gelöst Das Gerät ist hierbei üblicherweise so konzipiert, daß die mRNA hierbei nicht mit RNasen kontaminiert werden kann. Zur Lösung der mRNA wird üblicherweise ein geeigneter Puffer verwendet, der einerseits gute Transfektionseigenschaften aufweist und daneben sich durch eine hohe Löslichkeit für mRNA auszeichnet, in Frage kommt üblicherweise zum Beispiel folgender Puffer: 150 mM NaCl, 10 mM HEPES, pH 7,4. Dieser Puffer ist in dem Injektionsgerät üblicherweise schon enthalten oder kann vorzugsweise vor Injektion aus einem sterilen, RNase freien Gefäß aufgezogen werden.

Sämtliche Materialien dürfen vorzugsweise keine Kontaminationen mit RNasen aufweisen. Auch beim weiteren Gebrauch dürfen vorzugsweise keine RNasen in das Gerät eindringen. Dies kann vorzugsweise durch eine geeignete Verpackung des Geräts bewerkstelligt werden sowie vorzugsweise durch einen Schutz der Ein- und Austritts-Öffnungen.

Die Aufbewahrung des Lösungsmittels (Puffers) für die mRNA wird üblicherweise am einfachsten dadurch bewerkstelligt, das ein Pufferreservoir (3) beispielsweise an einem Stempel (2) angebracht ist. So kann beispielsweise durch Penetration einer Perforationsstelle (4) des Reservoirs der Puffer in den Impfstofflösungsraum (10) gelangen und die mRNA auflösen. Der Puffer könnte auch Ober die Injektionsnadel aus einem externen Gefäß aufgezogen werden, wie es für andere gebräuchliche Impfstoffe üblich ist, hierbei besteht aber die Gefahr, das Gerät mit mikrobiologischen Keimen oder RNasen zu kontaminieren.

Zur besseren Veranschaulichung kann bspw. die erfindungsgemäße Vorrichtung, ein mRNA-Injektionsgerät, folgendermaßen funktionieren: Durch Druck auf den Stempel (2) wird das Pufferreservoir (3) gegen ein Öffnungsmittel (7), eine Perforationsnadel (7), gedrückt .Diese Perforationsnadel kann - wie hier - aus einer Verlängerung der Injektionsnadel (6) bestehen. Durch die Perforation des Pufferreservoirs kann der Puffer in den Raum mit dermRNA, den Impfstofflösungsraum (10) gelangen. Das Einfließen des Puffers in den Impfstofflösungsraum erfolgt hier aktiv durch Ziehen des Stempels (2). Ebenso muß hier die Möglichkeit eines Druckausgleichs bestehen. Der Eintritt von Luft kann durch eine Öffnung, ein Ventil (1), welches oberhalb der Stempels(2) angebracht ist, erfolgen. Um vor Kontaminationen zu schützen, kann - wie hier - ein Luftfilter an diesem Ventil angebracht sein. Je nach Beschaffenheit der mRNA-Matrix (5) bzw. des mRNA-Pulvers kann sich die mRNA nun im Puffer im Impfstofflösungsraum lösen. Das kann auch durch Bewegung des Gerätes, wie z.B. schütteln, beschleunigt werden. Durch Verschließen des Ventils (1) ist hier das Innere des Gerätes vor Kontaminationen geschützt.

Die Impfstofflösung wäre gemäß dem obigen Beispiel nun applikationsfertig und könnte nun direkt zum Einsatz kommen. Hierzu kann bspw. die Schutzhülle (9) von der injektionsnadel (7) gelöst und die Nadel in das zu injizierende Gewebe gesetzt werden. Durch Druck auf den Stempel (2) kann nun die mRNA sicher und kontaminationsfrei appliziert werden. Dabei sorgt die Form und Art der Perforationsstelle (6) dafür, daß kein Puffer bzw. keine mRNA im Puffer in das Pufferreservoir zurückfließen kann. Um die meist kleinen Volumina (ca. 0,5 ml) vollständig applizieren zu können, wird z.B. an das Gerät eine Einbuchtung am inneren Ansatz der Injektionsnadel eingefügt, benannt als "Flüssigkeitssammler" (8), In den ein passendes Gegenstück am Stempel paßt. So kann bei vollständigem Pressen des Stempels die gesamte Flüssigkeit in die Injektionsnadel gedrängt

Ein weiterer Gegenstand der Erfindung ist die Verwendung dehydratisierter mRNA zur Herstellung einer Impf - oder Immunisierungsvorrichtung, dadurch gekennzeichnet, dass die mRNA auf mindestens einer, vorzugsweise mehreren, Hauptpenetrations -.

Dabei versteht man typischerweise vorrichtung(en) aufgebracht wird unter Impfstoff (gleichbedeutend mit Vakzine) eine Lösung oder Suspension, vorzugsweise eine Antigenlösung oder -suspension zur aktiven Immunisierung gegen Krankheiten, insbesondere Infektionskrankheiten. Unter Immunisierung versteht man hier insbesondere die aktive Immunisierung, das Herbeiführen einer Immunität durch direkten Kontakt des Organismus mit dem Antigen, wobei Immunität die durch das Auftreten spezifischer Antikörper gekennzeichnete veränderte Reaktionsbereitschaft des Immunsystems gegenüber spezifischen Antigenen ist. Unter Impfung versteht man entsprechend das Einbringen eines entsprechenden Impfstoffes in den Körper.

Durch den Entzug von Wasser (Dehydratisierung) wird die Stabilität des Impfstoffes und damit die Wirkung der Impfung überhaupt verbessert. Im trockenen Zustand ist die mRNA nicht mehr durch RNasen angreifbar und es kann keine molekülspezifische Hydrolyse stattfinden.

Vorteilhaft ist die Verwendung dehydratisierter mRNA, wenn die verwendete dehydratisierte mRNA nicht komplexiert ist, nicht auf einem Träger bzw. einer Matrix, wie z.B. Micropartikeln, beispielsweise aus Metall, gebunden ist, nicht infektiös ist und/oder nicht replikativ ist. Weiter vorteilhaft wäre die direkte Verwendung der dehydratisierten/getrockneten mRNA unter Umgehung der Oberhaut, beispielsweise an einer künstlichen Öffnung in der Haut, wie z.B. einem Schnitt oder anderen Wunde.

Besonders bevorzugt ist auch eine erfindungsgemäße Verwendung, bei der die Vorrichtung mit mindestens einer, vorzugsweise mehreren, insbesondere einer Anordnung mehrerer Hautpenetrationsvorrichtungen, vorzugsweise Nadeln, ausgestattet ist, wobei sich die getrocknete mRNA auf der/den Hautpenetrationseinrichtung/en befindet. Dabei kann entweder die mRNA in Lösung auf die Hautpenetrationseinrichtung/en aufgebracht und dort getrocknet werden oder die getrocknete mRNA auf die Hautpenetrationseinrichtung/en aufgebracht werden. Damit ist es möglich die getrocknete mRNA intracutan oder intradermal zu applizieren. Besonders bevorzugt wäre es auch, wenn zusammen mit der mRNA auf der/den Hautpenetrationseinrichtung/en (Nadel/n) auch Adjuvantien aufgebracht werden. Vor der Penetration kann z.B. ein Gel auf die Haut aufgebracht werden, um die RNA beim Eindrücken besser zu lösen. Bspw. kann auch die dehydratisierte mRNA vor der, vorzugsweise intradermalen oder intracutanen, Applikation auf mindestens einer Hautpenetrationseinrichtung, vorzugsweise einer Nadel, aufgebracht werden.

Die vorrichtung kann zur Immunisierung oder Impfung eines Lebewesens, insbesondere eines Säugetiers inklusive des Menschen, mit dehydratisierte mRNA verwendet werden.

Dazu kann bspw. die dehydratisierte mRNA vor der Injektion in Lösung gebracht werden.

Vorteilhaft ist es, wenn die verwendete dehydratisierte mRNA nicht komplexiert Ist, nicht auf einem Träger bzw. einer Matrix, wie beispielsweise Micropartikeln, beispielsweise aus Metall, gebunden ist, nicht infektiös ist und/oder nicht replikativ ist. Weiter vorteilhaft wäre direkte Verwendung der dehydratisierten/getrockneten mRNA (ebenfalls vorzugsweise ohne Kopplung an Träger oder Partikel) unter Umgehung der Oberhaut, beispielsweise an einer künstlichen Öffnung in der Haut, wie z.B. einem Schnitt oder anderen Wunde, insbesondere nach punktueller Entfernung von Hautschichten, einschließlich des sog. Stratum comeum, um die Barriere der Haut zu überwinden.

Die in einer erfindungsgemäßen Vorrichtung oder einer erfindungsgemäßen Verwendung eingesetzte mRNA weist vorzugsweise eine 5'-Cap-Struktur, eine Ihres ("Internal Ribosomal Entry Site") und/oder eine 3'-gelegene Stabilisierungssequenz auf. Auf diese Weise kann die Stabilität des als Vakzine eingesetzten mRNA-Materials erhöht und/oder die die Translationsfrequenz durch verstärkte Bindung von Ribosomen gesteigert werden. Darüber hinaus kann die erfindungsgemäß eingesetzte mRNA neben der Sequenz für ein antigenisches Protein/Peptid auch mindestens einen weiteren funktionalen Abschnitt enthalten, bspw. für ein die Immunantwort beförderndes Cytokin (Monokin, Lymphokin oder Interleukin oder Chemokin; bspw. IL-1, IL-2, IL-3, IL-4, IL-5, IL-6, IL-7, IL-8, IL-9, IL-10, IL-12, INF-a, INF-g, GM-CSF, LT-a oder Wachstumsfaktoren (bspw. GH)).

In ihrer Verwendung als Vakzine kommen die eingesetzten mRNA-Moleküle insbesondere zur Behandlung (bzw. zur Herstellung eines Arzneimittels) zur Behandlung von Krebserkrankungen (insbesondere mit Hilfe von mRNA, die für tumorspezifische Oberflächen-Antigene (TSSA) codiert), insbesondere zur Behandlung von malignem Melanom, Colon-Carcinom, Lymphomen, Sarkomen, kleinzelligem Lungencarcinom, Blastomen) in Betracht. Auch infektionserkrankungen (z.B. virale Infektionserkrankungen (z.B. HIV, Hepatits A, B oder C, Herpes, Varizellen (Herpes Zoster), Rubeolavirus (Röteln). Flaviviren (Gelbfieber, Dengue), Grippe (Influenzaviren), Marburg- oder Ebolaviren), bakterielle Infektionserkrankungen (Leglonärskrankheit (Legionellae), Magengeschwür (Helicobacter), Cholera (Vibrio). Infekte, die auf E.coli-Infektionen, Staphylokokken-Infektionen, Salmonellen-Infektionen oder Streptokokken-infektionen beruhen, Tetanus) oder protozoologische Infektionserkrankungen (Malaria, Schlafkrankheit, Chagas-Krankheit, Leishmaniose. Toxoplasmose, also Oberflächen-Antigene von Plasmodium, Trypanosoma, Leishmania, Toxoplasma) sind unter Einsatz der erfindungsgemäßen Vorrichtungen, oder Verwendungen therapierbar. Vorzugsweise werden auch im Falle von Infektionserkrankungen die entsprechenden Oberflächenantigene mit dem stärksten antigenen Potential zur Vakzinierung eingesetzt.

Wie zuver beschrieben, ist eine vorrichtung besonders vorteilhaft, die mit mindestens einer, vorzugsweise mehreren, insbesondere einer Anordnung mehrerer Hautpenetrationsvorrichtungen, vorzugsweise Nadeln, ausgestattet ist, wobei sich die getrocknete mRNA auf der/den Hautpenetrationseinrichtung/en befindet Dabei kann entweder die mRNA in Lösung auf die Hautpenetrationseinrichtung/en aufgebracht und dort getrocknet werden oder die getrocknete mRNA auf die Hautpenetrationseinrichtung/en aufgebracht werden. Damit ist es bspw. möglich, die getrocknete mRNA intracutan oder intradermal zu applizieren. Es können zusammen mit der mRNA auf der/den Hautpenetrationseinrichtung/en (Nadel/n) auch Adjuvantien aufgebracht werden. Ebenfalls kann vor der Penetration ein Gel auf die Haut aufgebracht werden um die mRNA beim Eindrücken besser zu lösen.

Im folgenden Abschnitt wird die Erfindung weiter durch Beispiele erläutert ohne sie darauf zu beschränken.

### Beispiele und Figuren:

### Figuren:

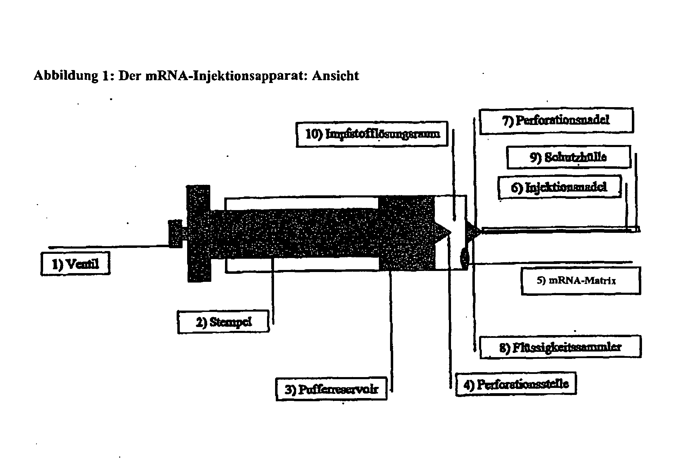

Die Figuren 1-5 zeigen beispielhafte mRNA-Injektionsapparate, die nicht unter den Schulzbereich der Ansprüche fallen, aus Verständnisgründen jedoch beigefügt sind.

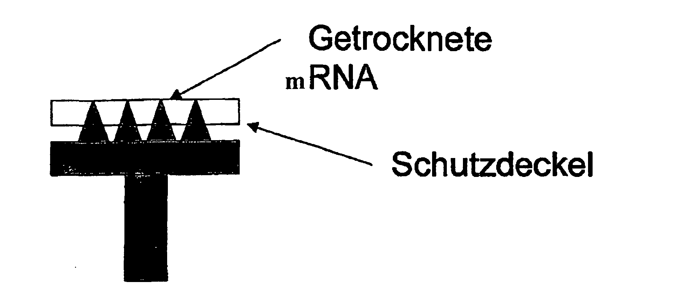

**Figur 1 (Fig. 1/10)** zeigt ein Beispiel eines mRNA-Injektions apparates gemäß Beispiel 6 in einer Gesamtansicht. Dabei bedeuten:
   1) Ventil,
   2) Stempel,
   3) Pufferreservoir,
   4) Perforationsstelle,
   5) mRNA-Matrix,
   6) Injektionsnadel,
   7) Perforationsnadel,
   8) Flüssigkeitssammler,
   9) Schutzhülle,
   10) Impfstofflösungsraum.

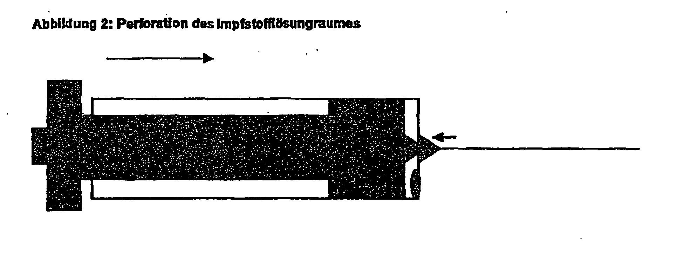

**Figur 2 (Fig. 2/10)** zeigt bei einem beispielhaften mRNA-Injektionsapparat gemäß Beispiel 6 die mögliche Herstellung der Impfstofflösung (Schritt 1). Demonstriert wird der erste Schritt, die Perforation des Pufferreservoirs (3). Durch eine Bewegung des Stempels in Richtung Perforationsnadel (7) erreicht diese die Perforationsstelle (4) und durchstößt sie, so daß ein Austritt des Puffers und ein Einströmen des Puffers aus dem Pufferreservoir (3) in den Impfstofflösungsraum (10) Ober die Perforationsstelle (4) ermöglicht wird.

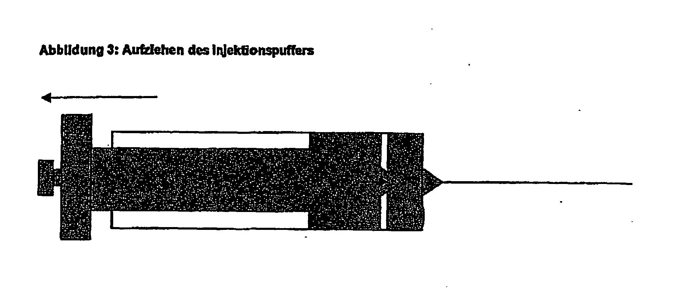

**Figur 3 (Fig. 3/10) zeigt bei einem beispielhaften** mRNA-Injektionsapparat gemäß Beispiel 6 die mögliche Herstellung der Impfstofflösung (Schritt 2). Demonstriert wird das Aufziehen des Injektionspuffers. Durch eine Bewegung des Stempels (2) in Pfeilrichtung (weg von der Perforationsnadel(7)) und einen Druckausgleich Ober das mit einem Filter versehene Ventil (1) kann der Puffer dem Druckgefälle folgend Ober die perforierte Perforationsstelle (4) aus dem Pufferreservoir (3) in den Impfstofflösungsraum (10) einströmen.

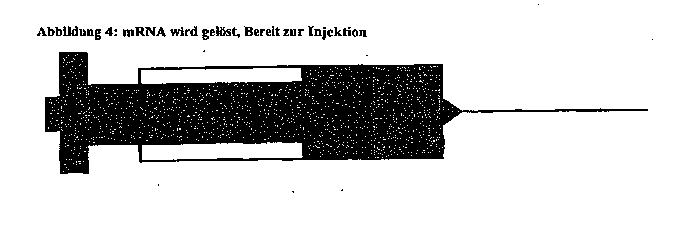

**Figur 4 (Fig. 4/10)** zeigt bei einem beispielhaften mRNA-Injektionsapparat gemäß Beispiel 6 die mögliche Herstellung der Impfstofflösung (Schritt 3). Demonstriert wird die Lösung der mRNA. Durch das Einströmen des Puffers in den Impfstofflösungsraum (10) wird die mRNA. Matrix (5) mit dem Puffer in Verbindung gebracht, so daß sich die mRNA im Injektionspuffer lösen kann.

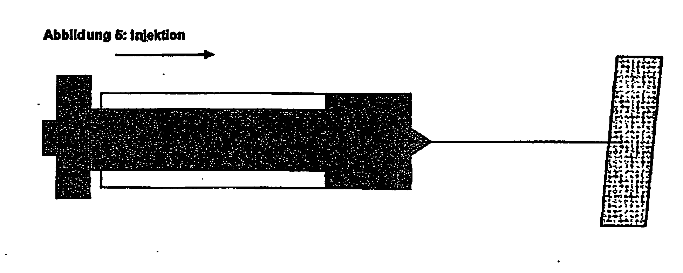

**Figur 5 (Fig. 5/10)** zeigt bei einem beispielhaften mRNA-Injektionsapparat gemäß Beispiel 6 die intradermale Injektion. Durch eine Bewegung des Stempels (2) wird die im Injektionspuffer gelöste mRNA über die Injektionsnadel (6) in das Zielgewebe eingebracht, nachdem die Schutzhülle (9) entfernt wurde. Durch den Flüssigkeitssammler (8), eine Einbuchtung am inneren Ansatz der Injektionsnadel, in den ein passendes Gegenstück am Stempel (2) paßt, wird bei vollständigem Pressen des Stempels (2) die gesamte Flüssigkeit in die Injektionsnadel (6) gedrängt.

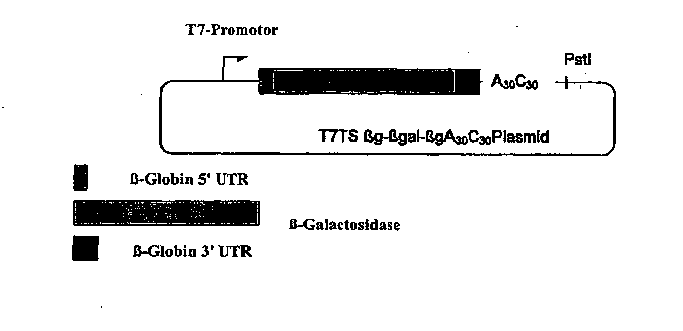

**Figur 6 (Fig. 6/10)** ist ein Schema zu Beispiel 1 und zeigt ein Pstl linearisiertes T7TS βg-βgal-βgA₃₀C₃₀ Plasmid sowie die UTRs und die β-Galactosidase.

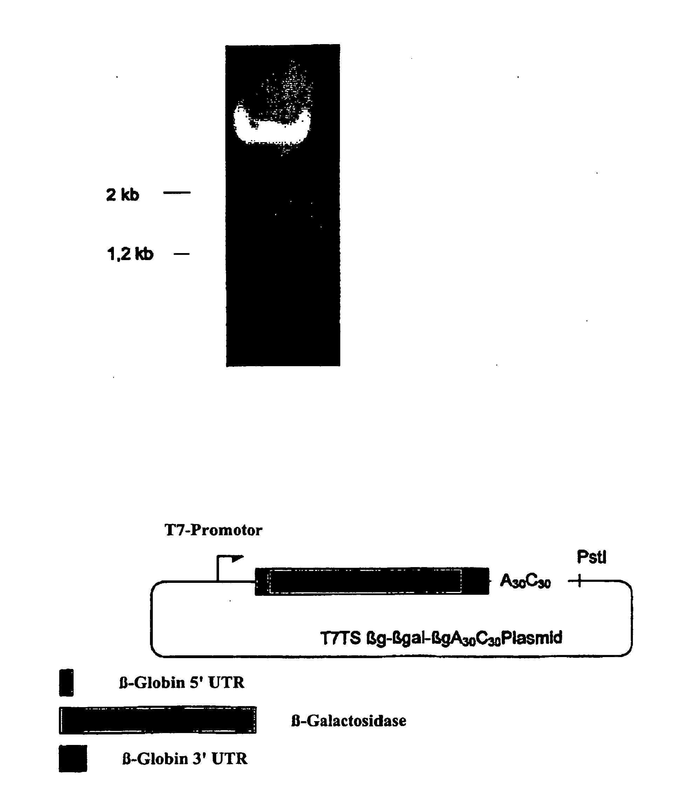

**Figur 7 (Fig. 7/10):** Auswertung von Beispiel 1. Das mit Ethidiumbromid gefärbte Agarosegel beweist die Integrität der 3,4 kb βg-βgal-βgA₃₀C₃₀-mRNA.

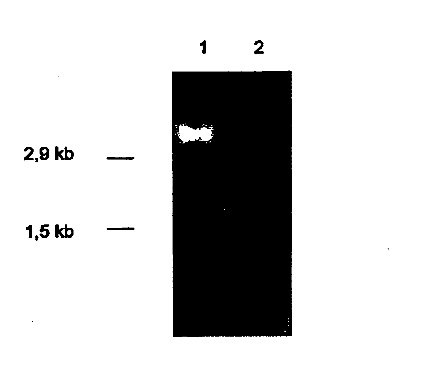

**Figur 8 (Fig. 8/10):** Auswertung von Beispiel 4. Ein mit Ethidiumbromid gefärbtes Agarosegel beweist die Integrität der 3,4 kb βg-βgal-βgA₃₀C₃₀-mRNA, die trocken für 3 Wochen auf Silika-Perlen aufbewahrt worden war. Spalte 1 entspricht 3 µg der βg-βgal-βgA₃₀C₃₀-mRNA, Spalte 2: 10 µl der in 200 µl Puffer von den Silika-Perlen eluierten mRNA.

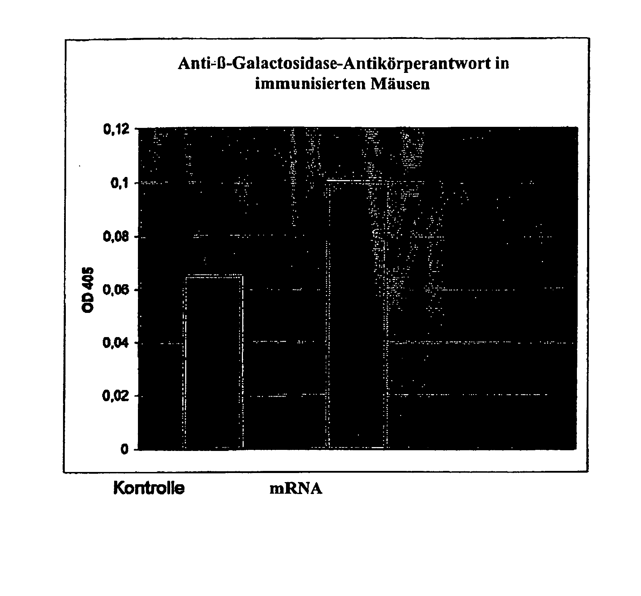

**Figur 9 (Fig. 9/10):** Auswertung von Beispiel 5. Detektion von Anti-βgalactosidase-Antikörpern (IgG2a) im Blut von Mäusen, die mit 100 µl Injektionspuffer (Kontrolle) und mit 100 µl Injektionspuffer mit dehydratisierter/getrockneter mRNA, die auf Silika-Perlen aufbewahrt worden war und vor der Injektion in Injektionspuffer resuspendiert wurde (mRNA), intradermal behandelt wurden.
**Figur 10 (Fig. 10/10):** Impfung unter direkter Verwendung getrockneter mRNA. Gezeigt wird ein Multitest-Immignost Stempel der Fa. Biosyn, mit dem die getrocknete mRNA unter die Haut gebracht wird, die in trockener Form auf kleinen Nadeln aufliegt Auf die Nadeln wird ein RNasefreier Deckel aufgelegt (s. Beispiel 9).

### Beispiel 1 :

### Herstellung einer spezifischen mRNA

Eine mRNA (Länge: 3.4 kb), die für E. coli β-Galactosidase codiert und β-Globin 5' und 3' "Untranslated Stabilisation Sequences" (UTRs) wurde in vitro durch Transkription des Pstl linearisierten T7TS βg-βgal-βgA₃₀C₃₀ Plasmids (s.u.) unter Verwendung des "T7 CAP Scribe Kit" (Roche) hergestellt (s. Figur 6).

Nach DNase-Behandlung wurde die mRNA-enthaltende Lösung mit einem gleichen Volumen einer Phenol/Chloroform/Isoamylalkohol-Mischung (zur Eliminierung der Proteine) versetzt und durch Zugabe von 4 Volumenanteilen einer Ethanol/7.5M Ammoniumacetat-Mischung (6/1 vol/vol) zur wässrigen Phase, Mischen und Zentrifugation über 15 Minuten bei 13000 rpm in einer Microzentrifuge präzipitiert Der überstand wurde verworfen und das mRNA-Pellet mit 1 ml 75% Ethanol gewaschen. Das Eppendorfgefäß wurde dann wieder 15 Minuten bei 13000 rpm zentrifugiert und der Überstand entfernt Das Pellet wurde 15 Minuten an der Luft getrocknet und bei einer mRNA-Endkonzentration von 1 mg/ml in Wasser resuspendiert. 1 µl wurde auf einem Agarosegel analysiert (s. Abb. 6). Das mit Ethidiumbromid gefärbte Agarosegel zeigt die Integrität der 3,4 kb βg-βgal-βgA₃₀C₃₀mRNA (Fig. 6).

### Beispiel 2:

**Herstellung eines gefriergetrockneten mRNA-Pulvers entsprechend** WO 95/27721

Es wurde eine Stabilisierungsmischung aus 15% w/w Saccharose, 5% w/w Mannitol und 5% w/w Dextran T-40 in Wasser hergestellt. 50 ml der Stabilisierungsmischung wurden mit 50 ml der 1:50 mit Wasser verdünnten mRNA-Lösung nach Beispiel 1 gemischt.

Die Lyosphären wurden wie folgt aus dieser mRNA/Stabilisator-Mischung hergestellt:

50µl-Portionen dieser Mischung wurden In kochenden Stickstoff getropft und darin gelassen, bis sie komplett gefroren waren. Die gefrorenen Sphären wurden dann nach üblichen Verfahren lyophilisiert, so daß die Sphären ein Restwassergehalt unter 2% w/w enthielten.

### Beispiel 3:

### Herstellung und Lagerung von mit mRNA-beschichteten Perlen

30 µl der mRNA nach Beispiel 1 wurde mit 20 µl Qiaex 11 Suspension (enthaltend Silika-Kugeln) und 100 µl eines Waschpuffers (Puffer PE, "Qiaex II Gel extraction kit". Qiagen) in einem Eppendorfröhrchen versetzt. Das Röhrchen wurde 1 Minute bei 13000 rpm in einer Microzentrifuge zentrifugiert. Der Überstand wurde verworfen und die Perlen 15 Minuten an der Luft getrocknet Die auf den Perlen immobilisierte getrocknete mRNA wurde bei Raumtemperatur und in der Dunkelheit drei Wochen aufbewahrt.

### Beispiel 4:

### Wiederablösung der mRNA von den mit mRNA-beschichteten Perlen

200 µl Injektionspuffer (Hepes pH 7.5 10mM, NaCl 150 mM) wurden zu den Perlen hinzugefügt Das Eppendorfgefäß wurde bei Raumtemperatur 15 Minuten inkubiert und dann bei 13000 rpm 1 Minute in einer Microzentrifuge zentrifugiert Der Überstand (und nicht die Perlen) wurde weiterverwendet. 10 µl wurden auf einem Agarosegel untersucht
Ein mit Ethidiumbromid gefärbtes Agarosegel beweist die Integrität der 3,4 kb βg-βgal-βgA₃₀C₃₀-mRNA. die trocken für 3 Wochen auf Sllika-Perlen aufbewahrt worden war (Figur 8).
Hiermit ist bewiesen, daß mRNA trocken für 3 Wochen auf Silika-Perlen aufbewahrt und dann nach Elution für eine Impfung eingesetzt werden kann. Die bei Raumtemperatur auf Silika-Perlen getrocknete/dehydratisierte mRNA war nicht abgebaut worden (Figur 8).

### Beispiel 5:

### Immunisierung/Impfung von Mäusen

100 µl des in Beispiel 4 und Figur 8 untersuchten Produkts von Beispiel 4 wurden einer BALB/c Maus intradermal injiziert.

Zwei Monate nach der mRNA-Injektion wurde das Blut der Mäuse durch ELISA auf seinen Gehalt an Anti-β-Galactosidase-Immunoglobulin untersucht. Dazu wurden ELISA-Platten mit β-Galactosidase-Proteinen beschichtet und mit BSA abgesättigt. Das Serum der Mäuse wurde den Wells zugesetzt. Die Platten wurden 4 Stunden bei Raumtemperatur aufbewahrt und viermal mit Phosphate-buffered Saline(PBS)/0.05% Tween gewaschen. Verdünnter Antimaus-IgG2a HRP-assoziierter Antikörper wurde den Wells für 2 Stunden beigegeben. Die Platten wurden viermal mit Phosphate-buffered Saline/0.05% Tween gewaschen. Dann wurden jedem Well 100 µl ABTS-Substrat (150 mg 2,2'-Azino-bis-(3-ethylbenzthiazolin-6-Sulfonsäure) in 500 ml 0,1 M Zitronensäure, pH eingestellt auf 4,35 mit NaOH) zugegeben. Nach wenigen Minuten Inkubationszeit wurde die OD₄₀₅ gemessen. Die bei Raumtemperatur auf Silika-Perlen getrocknete/dehydratisierte mRNA nach Beispiel 4 konnte nach der Elution eine deutliche Anti-βgalactosidase-Immunantwort auslösen, wenn sie intradermal in Mäuse injiziert wurde (s. Figur 9). Bei der Detektion von Anti-βgalactosidase-Antikörpern (lgG2a) im Blut von Mäusen, die mit 100 µl Injektionspuffer (Kontrolle) und mit 100 µl Injektionspuffer mit dehydratisierter/getrockneter mRNA, die auf Silika-Perlen aufbewahrt worden war und vor der Injektion in Injektionspuffer resuspendiert wurde (mRNA), intradermal behandelt wurden, zeigt sich, daß die Impfung mit der getrockneten mRNA erfolgreich war.

Die folgenden Beispiele 6-8 fallen nicht unter den Schutzbereich der Ansprüche, sie sind jedoch aus Verständinsgründen begefügt.

### Beispiel 6:

### Ein beispielhafter mRNA-Injektionsapparat, ein Gerät mit getrockneter/dehydratisierter mRNA gemäß einer der Figuren 1) bis 5) beispielsweise zur Impfung.

Dabei bedeuten:
(1) Ventil,
(2) Stempel,
(3) Pufferreservoir,
(4) Perforadonsstelle,
(5) mRNA-Matrix,
(6) Injektionsnadel,
(7) Perforationsnadel,
(8) Flüssigkeitssammler,
(9) Schutzhülle,
(10) Impfstofflösungsraum.

Bei diesem mRNA-Injektionsapparat liegt die mRNA beispielsweise gemäß Beispiel 4 direkt im Injektionsgerät in stabilisierter Form vor. Diese applikationsfertige mRNA kann über einen längeren Zeitraum problemlos gelagert werden (s. Beispiel 4, Figur 8). Für einen Einsatz, beispielsweise zur Impfung, wird dehydratisierte mRNA über eine Matrix (5) (wie beispielsweise in den Beispielen 3) und 4)) im Injektionsgerät, ggf. unter gleichzeitigem Einsatz von getrockneten mRNA-Inhibitoren (bspw. auch einem mRNA-Inhibitor-Cocktail, aufbewahrt. Alternativ kann natürlich auch auf eine Matrix verzichtet werden und die dehydratisierte mRNA in Form eines gefriergetrockneten Pulvers (beispielsweise gemäß Beispiel 2) im Impfstofflösungsraum (1) vorliegen. Vor der Applikation wird die mRNA mit einem Injektionspuffer versetzt und gelöst. Das Gerät ist hierbei so konzipiert, daß die mRNA hierbei nicht mit RNasen kontaminiert werden kann. Zur Lösung der mRNA wird ein geeigneter Puffer verwendet, der einerseits gute Transfektionseigenschaften aufweist und daneben sich durch eine hohe Löslichkeit für mRNA auszeichnet In Frage kommt zum Beispiel folgender Puffer
150 mM NaCl,
10 mM HEPES, pH 7,4.

Dieser Puffer ist in diesem Injektionsgerät bereits am Stempel (2) anliegend als Pufferreservoir (3) bereits integriert, was vorteilhaft ist. Durch Penetration einer Perforationsstelle (4) des Reservoirs kann der Puffer in den Impfstofflösungsraum (10) gelangen und die mRNA auflösen. Alternativ kann - beispielsweise bei Fehlen des Pufferreservoir (3) - der Puffer vor Injektion aus einem sterilen, RNase-freien Gefäß aufgezogen werden, wie es für andere gebräuchliche Impfstoffe üblich ist. Hierbei besteht aber die Gefahr, das Gerät mit mikrobiologischen Keimen oder RNasen zu kontaminieren.

Grundsätzlich ist es absolut notwendig, auf den Schutz vor RNasen zu achten. Sämtliche Materialien dürfen keine Kontaminationen mit RNasen aufweisen. Auch beim weiteren Gebrauch dürfen keine RNasen in das Gerät eindringen. Dies kann und wird in diesem Beispiel durch eine geeignete Verpackung sowie durch einen Schutz der Ein- und Austritts-Öffnungen des Geräts erreicht.

Grundsätzlich funktioniert das mRNA-Injekdonsgerät folgendermaßen: Durch Druck auf den Stempel (2) wird das Pufferreservoir (3) gegen eine Perforationsnadel (7) gedrückt. Diese Perforationsnadel kann - wie hier - aus einer Verlängerung der Injektionsnadel (6) bestehen. Durch die Perforation des Pufferreservoirs kann der Puffer in den Impfstofflösungsraum gelangen. Das Einfließen des Puffers In den Impfstofflösungsraum erfolgt aktiv durch Ziehen des Stempels (2). Ebenso muß die Möglichkeit eines Druckausgleichs bestehen. Der Eintritt von Luft kann durch ein Ventil (1), welches oberhalb der Stempels(2) angebracht ist, endigen. Um vor Kontaminationen zu schützen, kann - wie hier - ein Luftfilter an diesem Ventil angebracht sein. Je nach Beschaffenheit der mRNA-Matrix (5) bzw. des mRNA-Pulvers kann sich die mRNA nun im Puffer im Impfstofflösungsraum lösen. Das kann auch durch Bewegung des Gerätes, wie z.B. schütteln beschleunigt werden. Durch Verschließen des Ventils (1) ist das Innere des Gerätes vor Kontaminationen geschützt.

Die Impfstofflösung ist nun applikationsfertig und kann nun direkt zum Einsatz kommen. Hierzu wird die Schutzhülle (9) von der Injektionsnadel (7) gelöst und die Nadel in das zu injizierende Gewebe gesetzt. Durch Druck auf den Stempel (2) kann nun die mRNA sicher und kontaminationsfrei appliziert werden. Dabei sorgt die Form und Art der Perforationsstelle (6) dafür, daß kein Puffer bzw. keine mRNA im Puffer in das Pufferreservoir zurückfließen kann. Um die meist kleinen Volumina (ca. 0,5 ml) vollständig applizieren zu können, wird an das Gerät eine Einbuchtung am inneren Ansatz der Injektionsnadel eingefügt, benannt als "Flüssigkeitssammler" (8), in den ein passendes Gegenstück am Stempel paßt So wird bei vollständigem Pressen des Stempels die gesamte Flüssigkeit in die Injektionsnadel gedrängt.

### Beispiel 7:

### Herstellung von mit mRNA-beschichteten Gold-Partikeln

30 µl der mRNA nach Beispiel 1 wurden mit 20 µl einer Suspension von Goldpartikeln in Waschpuffer und 100 µl eines Waschpuffers in einem Eppendorfröhrchen versetzt. Das Röhrchen wurde 1 Minute bei 13000 rpm in einer Microzentrifuge zentrifugiert. Der Überstand wurde verworfen und die Partikel mit der auf den Partikeln immobilisierte getrocknete RNA 15 Minuten an der Luft getrocknet

### Beispiel 8:

### Impfung von Mäusen mit einem PowderJect-Gerät

Mit getrockneter mRNA (bspw. β-Galactosidase) beschichtete Gold-Partikel gemäß Beispiel 7 wurden frei von Kontamination, insbesondere mit fremder mRNA oder RNase-Inhibitoren, in eine "Drug Cassette" für ein PowderJect-Gerät gemäß Angaben des Herstellers der PowderJect Pharmaceuticals plc. (s. z.B. Website) überführt und die Partikel mittels des Gas-Stoßes, insbesondere Heliumstoßes, in die Haut von Balb/c Mäusen überführt. Es war 5h nach der Vakzinierung Proteinexpression in der Hautpartie nachweisbar, ebenso wie gegen die exprimierten Proteine gerichtete IgG-Antikörper.

### Beispiel 9:

### Impfung unter direkter Verwendung getrockneter mRNA

Es wurde ein Multitest-Immignost Stempel der Fa. Biosyn verwendet, mit dem mRNA (s. Bsp. 1) unter die Haut gebracht wurde, die in trockener Form auf kleinen Nadeln auflag mRNA gemäß Beispiel 1 wurde direkt auf die Nadeln aufgetragen und dort zusammen mit Freund's Adjuvants getrocknet Auf die Nadeln wurde ein RNase-freier Deckel aufgelegt (s. Abildung 10).

Nach Impfung wurde die Proteinexpression und der Titer von gegen β-Galaktosidase gerichteten Antikörpern am Behandlungsort bestimmt Wiederum waren sowohl Proteinexpression als auch Antikörper-Titer (IgG) signifikant nachweisbar.

Vor der intracutanen Applikation am Mäuseohr und Meerschweinchenhaut wurde der Deckel entfernt und ein Gel auf die Haut aufgebracht, um die mRNA beim Eindrücken besser zu lösen.

## Patentansprüche

1. Vorrichtung zur intracutanen oder intradermalen Applikation getrockneter mRNA in einen Organismus, **dadurch gekennzeichnet, dass** die Vor-richtung mit mindestens einer, vorzugsweise mehreren, Hautpenetrationsvorrichtung(en) ausgestattet ist, wobei dehydratisierte, vorzugsweise gefriergetrocknete, mRNA auf der (den) Hautpenetrationsvorrichtung(en) aufgebracht ist.

2. Vorrichtung nach Anspruch 2, **dadurch gekennzeichnet, dass** die Vorrichtung eine Vakzinierungsvorrichtung ist.

3. Vorrichtung gemäß einem der Ansprüche 1 oder 2, **dadurch gekennzeichnet, dass** die dehydratisierte mRNA nichtinfektiös und/oder nichtreplikativ ist.

4. Vorrichtung gemäß einem der Ansprüche 1 bis 3, **dadurch gekennzeichnet, dass** die mRNA nicht mit anderen Molekülen komplexiert ist.

5. Vorrichtung gemäß einem der Ansprüche 1 bis 4, **dadurch gekennzeichnet, dass** die dehydratisierte mRNA - vorzugsweise-zum Zeitpunkt der Applikation der mRNA - nicht auf einer Matrix, insbesondere nicht auf Mikropartikel, aufgebracht ist.

6. Vorrichtung gemäß einem der Ansprüche 1 bis 5, **dadurch gekennzeichnet, dass** die Vorrichtung in allen Bereichen, die mit der dehydratisierten mRNA in Kontakt kommen, vorzugsweise vollständig, frei von Kontaminationen, insbesondere mit RNasen ist und/oder RNase-freies Material verwendet wird.

7. Vorrichtung nach einem der Ansprüche 1 bis 6, **dadurch gekennzeichnet, dass** die Hautpenetrationsvorrichtung(en) eine (mehrere) Nadel(n) ist (sind).

8. Vorrichtung nach einem der Anspruche 1 bis 7, **dadurch gekennzeichnet, dass** zusammen mit der mRNA Adjuvantien auf der (den) Hautpenetrationsvorrichtung(en) aufgebracht sind.

9. Vorrichtung nach einem der Ansprüche 1 bis 8, **dadurch gekennzeichnet, dass** auf die Hautpehetrationsvorrichtung(en) ein RNase-freier Deckel aufgelegt ist.

10. Vorrichtung nach einem der Ansprüche 1 bis 9, **dadurch gekennzeichnet, dass** die mRNA auf einer Anordnung mehrerer Hautpenetrationsvorrichtungen aufgebracht ist.

11. Verwendung von dehydratisierter, vorzugsweise gefriergetrockneter, mRNA zur Herstellung einer Impf- oder Immunisierungsvorrichtung, **dadurch gekennzeichnet, dass** die mRNA auf mindestens einer, vorzugsweise mehreren Hautpenetrationsvorrichtung(en) aufgebracht wird.

12. Verwendung nach Anspruch 11, **dadurch gekennzeichnet, dass** die mRNA auf einer Anordnung mehrerer Hautpenetrationsvorrichtung(en) aufgebracht wird.

13. Verwendung nach Anspruch 11 oder 12, **dadurch gekennzeichnet, dass** die mindestens eine Hautpenetfationsvorrichtung eine Nadel ist.

14. Verwendung nach einem der Ansprüche 11 bis 13, **dadurch gekennzeichnet, dass** die Vorrichtung zur intradermalen oder intracutanen Applikation der mRNA dient.

15. Verwendung nach einem der Ansprüche 11 bis 14, **dadurch gekennzeichnet, dass** zusammen mit der mRNA auf der (den) Hautpenetrationsvorrichtung(en) Adjuvantien aufgebracht werden.

## Claims

1. Device for intra-cutaneously or intra-dermally administering dried mRNA to an organism, **characterised in that** the device is provided with at least one, preferably several, skin penetration device(s), and dehydrated, preferably freeze-dried, mRNA is applied to the skin penetration devices(s).

2. Device as claimed in claim 2, **characterised in that** the device is a vaccination device.

3. Device as claimed in one of claims 1 or 2, **characterised in that** the dehydrated mRNA is non/infection and/or non-replicating.

4. Device as claimed in one of claims1 to 3, **characterised in that** the mRNA is not complexed with other molecules.

5. Device as claimed in one of claims 1 to 4, **characterised in that** the dehydrated mRNA is not applied to a matrix, in particular to microparticles - preferably at the instant the mRNA is administered.

6. Device as claimed in one of claims 1 to 5, **characterised in that** the device is preferably completely free of contamination in all areas which come into contact with the dehydrated mRNA, and in particular is used with RNases and/or RNase-free material.

7. Device as claimed in one of claims 1 to 6, **characterised in that** the skin penetration device(s) is (are) a (several) needle(s).

8. Device as claimed in one of claims 1 to 7, **characterised in that** adjuvants are applied to the skin penetration device(s) together with the mRNA.

9. Device as claimed in one of claims 1 to 8, **characterised in that** a RNase-free cover is fitted on the skin penetration device(s).

10. Device as claimed in one of claims 1 to 9, **characterised in that** the mRNA is applied to an arrangement of several skin penetration devices.

11. Use of dehydrated, preferably freeze-dried, mRNA for producing a vaccination or immunisation device, **characterised in that** the mRNA is applied to at least one, preferably several, skin penetration device(s).

12. Use as claimed in claim 11, **characterised in that** the mRNA is applied to an arrangement of several skin penetration devices.

13. Use as claimed in claim 11 or 12, **characterised in that** the at least one skin penetration device is a needle.

14. Use as claimed in one of claims 1 to 13, **characterised in that** the device is used for intra-dermally or intra-cutaneously administering mRNA.

15. Use as claimed in one of claims 11 to 14, **characterised in that** adjuvants are applied to the skin penetration device(s) together with the mRNA.

## Revendications

1. Dispositif pour l'administration cutanée ou intradermique d'ARNm (acide ribonucléique messager) déshydraté dans un organisme, **caractérisé en ce que** le dispositif est équipé d'au moins un, de préférence de plusieurs, dispositif(s) de pénétration de la peau, de l'ARNm déshydraté, de préférence lyophilisé, étant appliqué sur le (les) dispositif(s) de pénétration de la peau.

2. Dispositif suivant la revendication 1, **caractérisé en ce que** le dispositif est un dispositif de vaccination.

3. Dispositif suivant l'une des revendications 1 et 2, **caractérisé en ce que** l'ARNm déshydraté est non infectieux et/ou non réplicatif.

4. Dispositif suivant l'une des revendications 1 à 3, **caractérisé en ce que** l'ARNm n'est pas complexé à d'autres molécules.

5. Dispositif suivant l'une des revendications 1 à 4, **caractérisé en ce que** l'ARNm déshydraté - de préférence à l'instant de son administration - n'est pas appliqué sur une matrice, en particulier sur des micro-particules.

6. Dispositif suivant l'une des revendications 1 à 5, **caractérisé en ce que** le dispositif est exempt dans toutes les régions, qui entrent au contact de l'ARNm déshydraté, de préférence totalement, de contaminations, en particulier par des ribonucléases et/ou un matériau exempt de ribonucléases est utilisé.

7. Dispositif suivant l'une des revendications 1 à 6, **caractérisé en ce que** le (les) dispositif(s) de pénétration de la peau est (sont) une (plusieurs) aiguilles(s).

8. Dispositif suivant l'une des revendications 1 à 7, **caractérisé en ce que** des adjuvants sont appliqués avec l'ARNm sur le (les) dispositif(s) de pénétration de la peau.

9. Dispositif suivant l'une des revendications 1 à 8, **caractérisé en ce qu'**un couvercle exempt de ribonucléase est mis en place sur le (les) dispositif(s) de pénétration de la peau.

10. Dispositif suivant l'une des revendications 1 à 9, **caractérisé en ce que** l'ARNm est appliqué sur un agencement de plusieurs dispositifs de pénétration de la peau.

11. Utilisation d'ARNm déshydraté, de préférence lyophilisé, pour la fabrication d'un dispositif de vaccination ou d'immunisation, **caractérisée en ce que** l'ARNm est appliqué sur au moins un, de préférence plusieurs, dispositif(s) de pénétration de la peau.

12. Utilisation suivant la revendication 11, **caractérisée en ce que** l'ARNm est appliqué sur un agencement de plusieurs dispositifs de pénétration de la peau.

13. Utilisation suivant l'une des revendications 11 ou 12, **caractérisée en ce que** le au moins un dispositif de pénétration de la peau est une aiguille.

14. Utilisation suivant l'une des revendications 11 à 13, **caractérisée en ce que** le dispositif sert à l'administration intradermique ou cutanée de l'ARNm.

15. Utilisation suivant l'une des revendications 11 à 14, **caractérisée en ce que** des adjuvants sont appliqués avec l'ARNm sur le (les) dispositif (s) de pénétration de la peau.
